# EUROPÄISCHE PATENTANMELDUNG

(11) **EP 0 697 213 A1**
(43) Veröffentlichungstag der Anmeldung: **21.02.1996**
(21) Anmeldenummer: 95112185.4
(22) Anmeldetag: 03.08.1995
(51) Int. Cl.: A61K 7/32

(54) **Desodorierende kosmetische Mittel**

(30) Priorität: 19.08.1994 DE 4429467
(71) Anmelder: Beiersdorf Aktiengesellschaft, D-20245 Hamburg (DE)
(72) Erfinder: Röckl, Manfred, D-22880 Wedel/Holst (DE); Traupe, Bernd, D-22457 Hamburg (DE); Klier, Manfred, D-20521 Aumühle (DE); Wolf, Florian, D-20251 Hamburg (DE)

(57) **Zusammenfassung**

Verwendung eines Gemisches aus
(a) Dodecansäure (Laurinsäure),
(b) mindestens einer weiteren Fettsäure, gewählt aus der Gruppe der unverzweigten gesättigten Fettsäuren einer Kettenlänge von C₆ ₋ ₂₀, wobei mindestens eine der weiteren Fettsäuren eine Kettenlänge größer als C₁₂ besitzen muß,
(c) Glycerylmonocaprinat und/oder Glycerylmonocaprinat
(d) unter Verzicht auf ethoxylierte Glycerylfettsäureester und propoxylierte Glycerylfettsäureester
(e) im pH-Bereich unter 8 vorliegend.
als Wirkprinzip zur selektiven Reduzierung coryneformer Bakterien bzw. als Deowirkstoff.

## Beschreibung

Die vorliegende Erfindung betrifft kosmetische Desodorantien. Solche Formulierungen dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Es wurde überraschend gefunden, und darin liegt die Lösung all dieser Aufgaben, daß die Verwendung eines Gemisches aus
(a) Dodecansäure (Laurinsäure),
(b) mindestens einer weiteren Fettsäure, gewählt aus der Gruppe der unverzweigten gesättigten Fettsäuren einer Kettenlänge von C₆ ₋ ₂₀, wobei mindestens eine der weiteren Fettsäuren eine Kettenlänge größer als C₁₂ besitzen muß,
(c) Glycerylmonocaprinat und/oder Glycerylmonocaprinat
(d) unter Verzicht auf ethoxylierte Glycerylfettsäureester und propoxylierte Glycerylfettsäureester
(e) im pH-Bereich unter 8 vorliegend.
als Wirkprinzip zur selektiven Reduzierung coryneformer Bakterien bzw. die Verwendung eines Gemisches aus
(a) Dodecansäure (Laurinsäure),
(b) mindestens einer weiteren Fettsäure, gewählt aus der Gruppe der unverzweigten gesättigten Fettsäuren einer Kettenlänge von C₆ ₋ ₂₀, wobei mindestens eine der weiteren Fettsäuren eine Kettenlänge größer als C₁₂ besitzen muß,
(c) Glycerylmonocaprinat und/oder Glycerylmonocaprinat
(d) unter Verzicht auf ethoxylierte Glycerylfettsäureester und propoxylierte Glycerylfettsäureester
(e) im pH-Bereich unter 8 vorliegend.
als Deowirkstoff
den Nachteilen des Standes der Technik abhilft.

Im weiteren wird das der Erfindung innewohnende Gemisch aus (a) Laurinsäure und (b) mindestens einer weiteren Fettsäure gelegentlich der Einfachheit halber als das "erfindungsgemäße Fettsäuregemisch" bezeichnet oder mit ähnlichen Bezeichnungen versehen.

Obwohl erfindungsgemäß alle fünf Bedingungen von (a) bis (e) erfüllt sein müssen, werden die Bestandteile (a) und (b) aus praktischen Gründen gelegentlich zusammengefaßt. In einer besonderen Ausführungsform der vorliegenden Erfindung, zumal wenn (a) und (b) einer bestimmten Fettsäurefraktion entnommenwerden können, bilden sie nämlich gewissermaßen eine Einheit.

Aus der EP-OS 0 243 145 ist zwar eine antimikrobiell wirksame topische pharmazeutische Zusammensetzung bekannt, die laut den unabhängigen Ansprüchen 2 und 3 eine ternäre Mischung aus einem Glycerylfettsäureester, und/oder einem ethoxylierten Glycerylfettsäureester und/oder einem propoxylierten Glycerylfettsäureester, einer Mischung aus Fettsäuren und einem pharmazeutisch annehmbaren Träger bekannt, wobei die Fettsäuren aus einer ersten Fettsäure einer Kettenlänge von C₆₋₁₈ und einer zweiten Fettsäure einer Kettenlänge von C₆₋₁₈ bestehen.

Die Dodecansäure und andere erfindungsgemäße Fettsäuren werden zwar formal von diesen generischen Fettsäureformeln umfaßt. Die in dieser Schrift beschriebene antimikrobielle Wirkung wird jedoch allein der Wirkung der Glycerylfettsäureester, der ethoxylierten Glycerylfettsäureester und/oder der propoxylierten Glycerylfettsäureester zugeschrieben, welche zwingend allen dort beschriebenen Zusammensetzungen zugrunde liegen.

Diese Schrift weist in eine andere Richtung als die, die durch die vorliegende Erfindung gegeben wird. In überraschender Weise hat sich nämlich herausgestellt, daß ein Zusatz von Glycerylfettsäureestern, ethoxylierten Glycerylfettsäureestern und/oder propoxylierten Glycerylfettsäureestern die antimikrobielle Wirkung des erfindungsgemäßen Gemisches aus Laurinsäure und anderen Fettsäuren erheblich mindert, in manchen Fällen, bei bestimmten Konzentrationen sogar gänzlich aufhebt.

Dies trifft für alle Glycerylfettsäureester zu, außer für Glycerylmonocaprylat und Glycerylmonocaprinat. Zwar sind diese beiden Glycerylester in der EP-OS 0 243 145 erwähnt. Diese Schrift offenbart aber nur Pharmaka, wobei der Sinn deren Applikation eben nicht darin besteht, selektiv die Körpergeruch erzeugenden Mikroorganismen abzutöten, sondern gegen eine Vielzahl von Mikroorganismen anzugehen. Die EP-OS 0 243 145 führt den Fachmann für Körperdesodorantien also in doppelter Hinsicht in die Irre.

Die EP-OS 0 465 423 beschreibt eine pharmazeutische Zusammensetzung zur Bekämpfung von Mikroorganismen, welche im wesentlichen einen inerten Träger und einen aktiven Bestandteil umfaßt, welcher aus einer wirksamen Menge einer oder mehrerer Verbindungen, gewählt aus Fettsäuren einer Kettenlänge von C₄₋₁₄ und deren Monoglyceriden sowie der einfach oder mehrfach ungesättigten Fettsäuren einer Kettenlänge von C₁₄₋₂₂ und deren Monoglyceriden, besteht. Diese Schrift offenbart gleichfalls nur Pharmaka und keine Kosmetika. Einen Hinweis in die Richtung der vorliegenden Erfindung findet sich in auch dieser Schrift nicht.

Schließlich ist bekannt, Seifen, also Salze der erfindungsgemäßen Säuren in Kombination einzusetzen. Diese sind jedoch nicht erfindungsgemäß aktiv. In überraschender Weise entfalten die erfindungsgemäßen Gemische ihre vorteilhafte antimikrobielle und desodorierende kosmetische Wirkung nur im pH-Bereich unter 8.

Die desodorierende Wirkung der erfindungsgemäßen Gemische beruht in erster Linie auf deren selektiver Toxizität für grampositive, insbesondere coryneforme Bakterien. Diese werden als die für die Zersetzung des apokrinen Schweißes hauptsächlich verantwortlichen Keime angesehen. Da diese Gemische zugleich völlig unschädlich für den Menschen und andere Warmblüter sind, sind sie ideal für die Verwendung in kosmetischen Desodorantien geeignet.

Insbesondere war überraschend, daß, obwohl Laurinsäure, die im allgemeinen den Hauptbestandteil der erfindungsgemäßen Gemische darstellt, als bei sehr empfindlichen Personen schwach hautreizend eingeschätzt wird, die erfindungsgemäßen Gemische aber nicht einmal bei Probanden mit empfindlicher Haut auch nur die geringsten Reizerscheinungen zeigten.

Ferner war überraschend, daß die erfindungsgemäßen Gemische in synergistischer Weise gegen coryneforme Bakterien wirken.

In "Antiseptika", Teil 3, Untertitel "Antibakterielle, antifungielle und antivirale Antiseptik - ausgewählte Wirkstoffe", Gustav Fischer Verlag, Stuttgart, New York, 1987, Ss. 250 ff. wird dargestellt, daß zwar Laurinsäure gegen coryneforme Bakterien gut wirksam ist, die anderen C₆₋₂₀-Fettsäuren jedoch bestenfalls schwache Wirkung zeitigen.

Am angegebenen Ort wird ferner erwähnt, daß einige der erfindungsgemäß einzusetzenden Fettsäuren über gewisse mikrobizide Eigenschaften verfügen. Über ein synergistisches Zusammenspiel der einzelnen Fettsäurekomponenten miteinander sowie mit Glycerinmonocaprinat und/oder Glycerinmonocaprylat war bisher nichts bekannt.

Glycerylmonocaprylat und Glycerylmonocaprinat sind durch die allgemeine Formel 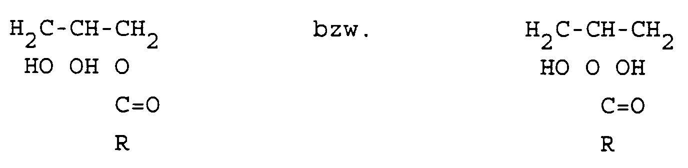
gekennzeichnet, wobei R den Heptanylrest und/oder Nonanylrest darstellen kann.

In dieser Schrift, insbesondere in den Beispielen, wird das Kürzel GMCy für Glycerinmonocaprylat und das Kürzel GMC für Glycerinmonocaprinat verwendet.

Bei den in 1-Position des Glycerins veresterten Glycerinestern ist die 2-Position ein Asymmetriezentrum. Erfindungsgemäß aktiv und gleichermaßen von Vorteil sind die 2S- und die 2R-Konfiguration.

Es hat sich als günstig herausgestellt, racemische Gemische der Stereoisomeren zu verwenden.

In den Endformulierungen, also den desodorierenden Kosmetika, beträgt der Gehalt an GMCy und/oder GMC vorteilhaft 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung.

Bevorzugt werden die C₆₋₂₀-Fettsäuren gewählt aus der Gruppe
Capronsäure (Hexansäure), Caprylsäure (Octansäure), Pelargonsäure (Nonansäure), Caprinsäure (Decansäure), Myristinsäure (Tetradecansäure), Palmitinsäure (Hexadecansäure) und Stearinsäure (Octadecansäure).

Es ist von Vorteil, folgende Gewichtsverhältnisse zu wählen, unabhängig, ob ein Gemisch der Laurinsäure mit einer oder mehreren weiteren Fettsäuren vorliegt:
Laurinsäure : Capronsäure = 50 : 0 bis 50 : 5 und/oder
Laurinsäure : Caprylsäure = 50 : 1 bis 50 : 20 und/oder
Laurinsäure : Pelargonsäure = 50 : 1 bis 50 : 20 und/oder
Laurinsäure : Caprinsäure = 50 : 1 bis 50 : 20 und/oder
Laurinsäure : Myristinsäure = 50 : 5 bis 50 : 30 und/oder
Laurinsäure : Stearinsäure = 50 : 1 bis 50 : 50,
Besonders vorteilhaft ist, folgende Gewichtsverhältnisse zu wählen:
Laurinsäure : Capronsäure = 50 : 0 bis 50 : 1 und/oder
Laurinsäure : Caprylsäure = 50 : 2 bis 50 : 15 und/oder
Laurinsäure : Pelargonsäure = 50 : 2 bis 50 : 15 und/oder
Laurinsäure : Caprinsäure = 50 : 2 bis 50 : 15 und/oder
Laurinsäure : Myristinsäure = 50 : 10 bis 50 : 20 und/oder
Laurinsäure : Stearinsäure = 50 : 2 bis 50 : 20.

Wenn Laurinsäure im Gemisch mit mehreren weiteren Fettsäuren vorliegt, so ist es ferner günstig, ein Gemisch aus Laurinsäure, Caprinsäure und Caprylsäure zu verwenden, besonders, wenn das Gewichtsverhältnis gewählt wird aus dem Bereich
Laurinsäure : Caprylsäure : Caprinsäure = 50 : 1 : 1 bis 50 : 20 : 20.

Bevorzugt wird dann das Gewichtsverhältnis gewählt aus dem Bereich
Laurinsäure : Caprylsäure : Caprinsäure = 50 : 1 : 1 bis 50 : 5 : 5.

Die Verwendung von Capronsäure ist zwar erfindungsgemäß durchaus vorteilhaft und führt an sich zu Gemischen mit guter Wirkung gegen coryneforme Bakterien. Wegen des unangenehmen Eigengeruches der Capronsäure sollte deren Konzentration jedoch niedrig gehalten werden.

Die bevorzugte Ausführungsform der vorliegenden Erfindung besteht darin, das Verhältnis der Laurinsäure zu einer oder auch mehreren der anderen erfindungsgemäßen Fettsäuren so zu wählen, daß es dem Verhältnis in einem hydrierten (gehärteten) Schnitt von Fettsäuren aus natürlichem Kokosfettsäurengemisch entspricht.

Die Zusammensetzung der natürlichen Kokosfettsäuren beträgt etwa:

| | |
|---|---|
| Laurinsäure | 44 - 51 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Ölsäure | 5 - 8 Gew.-% |
| Stearinsäure | 1 - 3 Gew.-% |
| Linolsäure | 0 - 2 Gew.-% |
| Capronsäure | 0 - 1 Gew.-% |

Die Zusammensetzung der gehärteten (hydrierten) Kokosfettsäuren beträgt etwa:

| | |
|---|---|
| Laurinsäure | 44 - 51 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 6 - 13 Gew.-% |
| Capronsäure | 0 - 1 Gew.-% |

Laurinsäure (CAS-No. 143-07-7) ist von verschiedenen Anbietern erhältlich, beispielsweise von der Aarhus Oliefabrik A/S, von der Aceto Chemical Company Inc., von der Dansk Sojakagefabrik A/S, von Procter & Gamble Ltd. und anderen.

Hydrierte Kokosnußfettsäure ist unter der Handelsbezeichnung Hydrofol^{R} Acid 631 von der Firma Ashland Chemical Company und unter der Bezeichnung Edenor^{R} HK 8-18 von der Firma Henkel KGaA erhältlich.

Bevorzugt ist folgende Zusammensetzung der erfindungsgemäßen Fettsäuregemische:

| | |
|---|---|
| Laurinsäure | 1 - 99 Gew.-% |
| Myristinsäure | 0 - 18 Gew.-% |
| Palmitinsäure | 0 - 10 Gew.-% |
| Caprylsäure | 0 - 9 Gew.-% |
| Caprinsäure | 0 - 10 Gew.-% |
| Stearinsäure | 1 - 99 Gew.-% |
| Capronsäure | 0 - 1 Gew.-% |

jeweils bezogen auf das Gesamtgewicht des Fettsäuregemisches.

Besonders vorteilhafte Zusammensetzungen werden erhalten, wenn die Zusammensetzung des erfindungsgemäßen Fettsäuregemisches wie folgt gewählt wird:

| | |
|---|---|
| Laurinsäure | 10 - 90 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 10 - 90 Gew.-% |
| Capronsäure | 0 - 1 Gew.-% |

jeweils bezogen auf das Gesamtgewicht des Fettsäuregemisches.

Insbesondere werden vorteilhafte Zusammensetzungen erhalten, wenn die Zusammensetzung des erfindungsgemäßen Fettsäuregemisches wie folgt gewählt wird:

| | |
|---|---|
| Laurinsäure | 44 - 51 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 6 - 13 Gew.-% |
| Capronsäure | 0 - 1 Gew.-%, |

jeweils bezogen auf das Gesamtgewicht des Fettsäuregemisches.

Laut Spezifikation zeichnet sich Edenor^{R} HK 8-18 durch folgenden ungefähren Gehalt an den erfindungsgemäßen Fettsäuren aus:

| | |
|---|---|
| Laurinsäure | 48 Gew.-% |
| Myristinsäure | 18 Gew.-% |
| Palmitinsäure | 8 Gew.-% |
| Caprylsäure | 7 Gew.-% |
| Caprinsäure | 7 Gew.-% |
| Stearinsäure | 10 Gew.-% |
| Capronsäure | 1 Gew.-% |

jeweils bezogen auf das Gesamtgewicht des Gemisches. Die Verwendung dieses Handelsproduktes ist vorteilhaft. In den Endformulierungen, also den desodorierenden Kosmetika, beträgt der Gehalt an den erfindungsgemäßen Fettsäuregemischen vorteilhaft 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung.

Das Verhältnis von GMCy und/oder GMC einerseits und dem Fettsäuregemisch andererseits zueinander kann vorteilhaft aus dem Bereich von 20 : 1 bis 1 : 20 gewählt werden, bevorzugt aus dem Bereich von 5 : 1 bis 1 : 5.

Die erfindungsgemäßen kosmetischen Desodorantien können in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, jedoch auch in Form von aus normalen Flaschen und Behältern auftragbaren W/O- oder O/W-Emulsionen, z.B. Crèmes oder Lotionen. Weiterhin können die kosmetischen Desodorantien vorteilhaft in Form von desodorierenden Tinkturen, desodorierenden Intimreinigungsmitteln, desodorierenden Shampoos, desodorierenden Dusch- oder Badezubereitungen, desodorierenden Pudern oder desodorierenden Pudersprays vorliegen.

Im allgemeinen sind desodorierende Gel-Stifte (Deo-Sticks) keine geeignete Darreichungsform für die erfindungsgemäßen Gemische, da sie zumeist hohe pH-Werte haben. Im basischen Milieu aber werden die erfindungsgemäßen Fettsäuregemische in die entsprechenden Seifen umgewandelt, welche nicht oder nicht genügend antimikrobiell wirksam sind.

Desodorierende Stifte, welche pH-Werte kleiner als 8 aufweisen, stellen dagegen durchaus vorteilhafte Darreichungsformen dar.

Als übliche kosmetische Trägerstoffe zur Herstellung der erfindungsgemäßen desodorierenden Mittel können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z.B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosiät.

Als Treibmittel für erfindungsgemäße, aus Aerosolbehältern versprühbare kosmetische Desodorantien sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorchlorkohlenwasserstoffe (FCKW).

Als Emulgatoren zur Herstellung der erfindungsgemäßen kosmetischen Desodorantien, welche vorteilhaft als flüssige Zubereitungen mittels einer Roll-on-Vorrichtung auf die gewünschten Hautbereiche aufgetragen werden sollen, und die in den Mitteln in geringer Menge, z.B. 2 bis 5 Gewichts.-%, bezogen auf die Gesamt-Zusammensetzung, verwendet werden können, haben sich nichtionogene Typen, wie Polyoxyethylen-Fettalkoholether, z.B. Cetostearylalkoholpolyethylenglykolether mit 12 bzw. 20 angelagerten Ethylenoxid-Einheiten pro Molekül, Cetostearylalkohol sowie Sorbitanester und Sorbitanester-Ethylenoxid-Verbindungen (z.B. Sorbitanmonostearat und Polyoxyethylensorbitanmonostearat) und langkettige höhermolekulare wachsartige Polyglykolether als geeignet erwiesen.

Zusätzlich zu den genannten Bestandteilen können den desodorierenden kosmetischen Mitteln gemäß der Erfindung, deren PH-Wert vorzugsweise z.B. durch übliche Puffergemische auf 4,0 bis 7,5 insbesondere 5,0 bis 6,5, eingestellt wird, Parfüm, Farbstoffe, Antioxidantien (z.B. alpha-Tocopherol und seine Derivate oder Butylhydroxytoluol (BHT = 2,6-Di-Tert.-butyl-4-methylphenol) in Mengen von 0,01 bis 0,03 %, bezogen auf die Gesamt-Zusammensetzung), Suspendiermittel, Puffergemische oder andere übliche kosmetische Grundstoffe beigemischt werden.

Es ist für die vorliegende Erfindung wesentlich, daß der pH-Wert der erfindungsgemäßen kosmetischen Desodorantien kleiner als 8 ist. pH-Werte, die leicht höher sind als 7, aber kleiner als 7,5 können dabei im allgemeinen toleriert werden. Jedenfalls ist für ein gegebenes Fettsäuregemisch im Einzelfalle durch einfaches Ausprobieren, ohne erfinderisches Dazutun, leicht zu ermitteln, welche exakte obere pH-Grenze zu beachten ist.

Die untere pH-Grenze wird allein von dermatologischen Gegebenheiten bestimmt. Da Kosmetika und Dermatika keinen pH-Wert kleiner als etwa 3,5 - 4 aufweisen sollten, kann dieser Bereich als untere Grenze angesehen werden. Dennoch sind die erfindungsgemäßen Zusammensetzungen grundsätzlich auch bei noch niedrigeren pH-Werten aktiv.

Vorteilhaft wird der pH-Wert der erfindungsgemäßen Formulierungen im sauren bis sehr schwach alkalischen Bereich kleiner als 8 eingestellt, bevorzugt von 4,0 - 7,5 , besonders bevorzugt von 5,0 - 6,5.

Die jeweils einzusetzenden Mengen an kosmetischen Trägerstoffen und Parfüm können in Abhängigkeit von der Art des jeweiligen Produktes vom Fachmann durch einfaches Ausprobieren leicht ermittelt werden.

Zur Parfümierung sind gegebenenfalls auch solche Substanzen und Parfümöle geeignet, die stabil sind, die Haut nicht reizen und bereits als solche antibakterielle oder bakteriostatische Eigenschaften besitzen.

Die Herstellung der kosmetischen Mittel erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Sie bietet keine Schwierigkeiten. Für Emulsionen werden Fettphase und die Wasserphase z.B. separat, gegebenenfalls unter Erwärmen hergestellt und dann emulgiert.

Ansonsten sind die üblichen Maßregeln für das Zusammenstellen von kosmetischen Formulierungen zu beachten, die dem Fachmann geläufig sind.

Sollen die erfindungsgemäßen Kombinationen in Pudersprays eingearbeitet werden, so können die Suspensionsgrundlagen dafür vorteilhaft gewählt werden aus der Gruppe Kieselsäuregele (z.B. solche die unter dem Handelsnamen Aerosil^{R} erhältlich sind), Kieselgur, Talkum, modifizierte Stärke, Titandioxid, Seidenpulver, Nylonpulver, Polyethylenpulver und verwandten Stoffen.

Es folgen vorteilhafte Ausführungsbeispiele der vorliegenden Erfindung.

### Beispiel 1

### Aerosolspray I

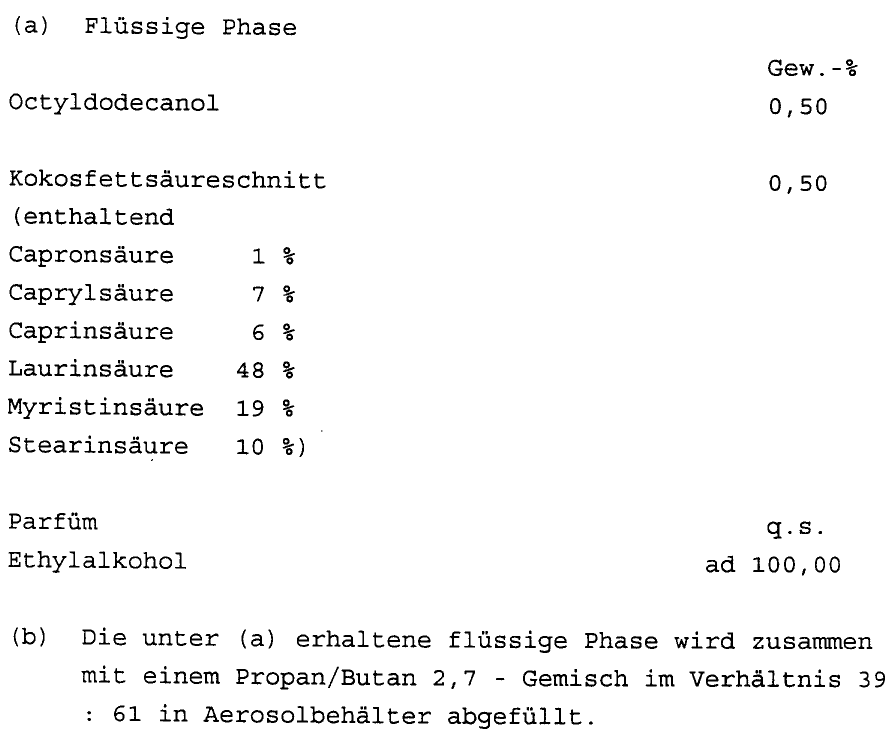

### Beispiel 2

### Aerosolspray II

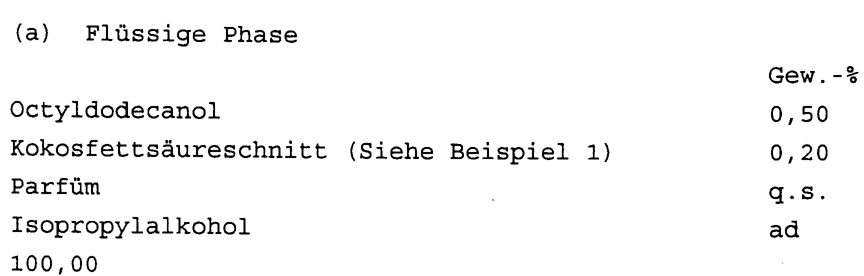

### Beispiel 3

### Pumpspray I

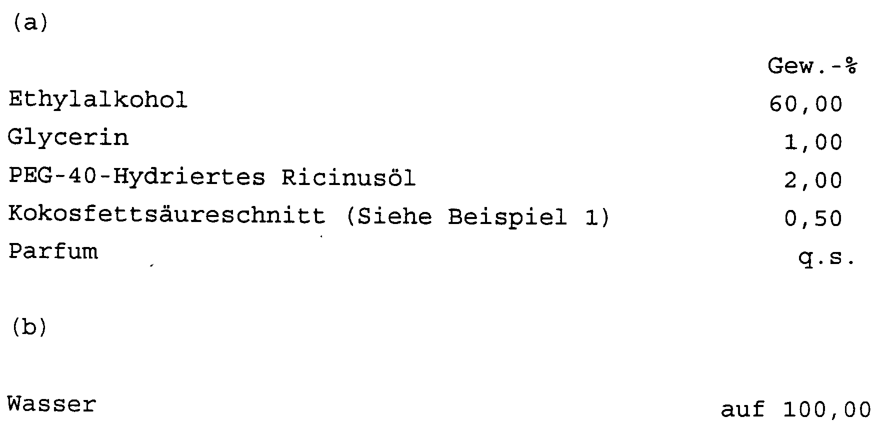

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 4

### Roll on - Gel I

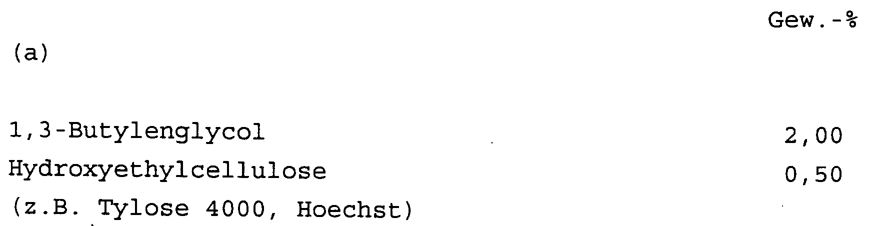

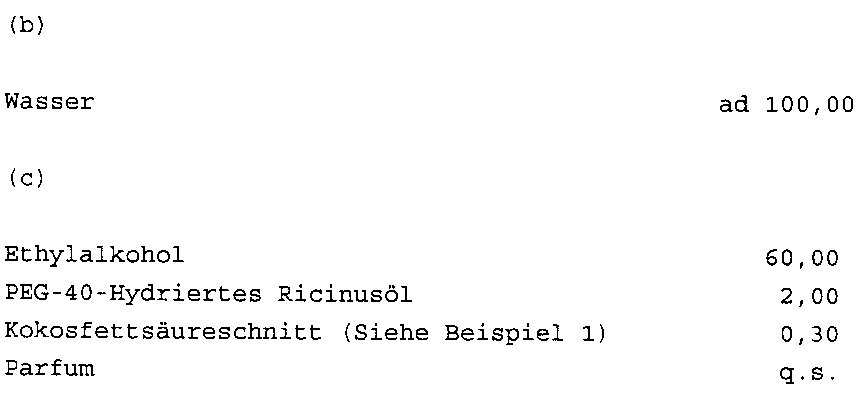

Die unter (a) genannten Bestandteile werden dispergiert, Wasser (b) wird zugegeben, bei Raumtemperatur quellen gelassen, eine Lösung der unter (c) genannten Bestandteile wird nach ca. 15 Minuten zugegeben. Die entstanden Mischung wird homogenisiert und kann abgefüllt werden.

### Beispiel 5

### Wachsstift I

| | Gew.-% |
|---|---|
| Hydriertes Ricinusöl | 5,00 |
| Bienenwachs | 6,00 |
| Ceresin (Hart-Ozokerit) | 30,00 |
| C₁₂₋₁₅-Alkohol-Benzoate | 17,00 |
| Kokosfettsäureschnitt (Siehe Beispiel 1) | 0,40 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 6

### Roll on - Emulsion I

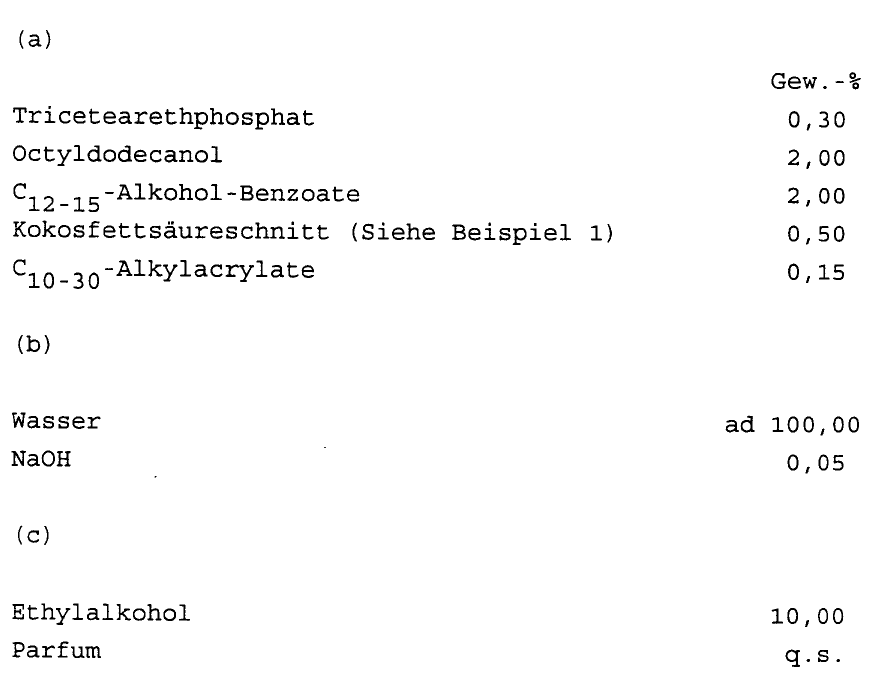

Die unter (a) und (b) genannten Bestandteile werden jeweils unter Rühren auf 75° C erwärmt. Sodann werden die Bestandteile (a) zu (b) gegeben. Die Mischung wird auf 35° C abgekühlt. Aus den Bestandteilen (c) wird eine Lösung hergestellt, welche auf 35° C erwärmt wird und unter Rühren zur Mischung aus (a) und (b) gegeben wird.

### Beispiel 7

### Aerosolspray IV

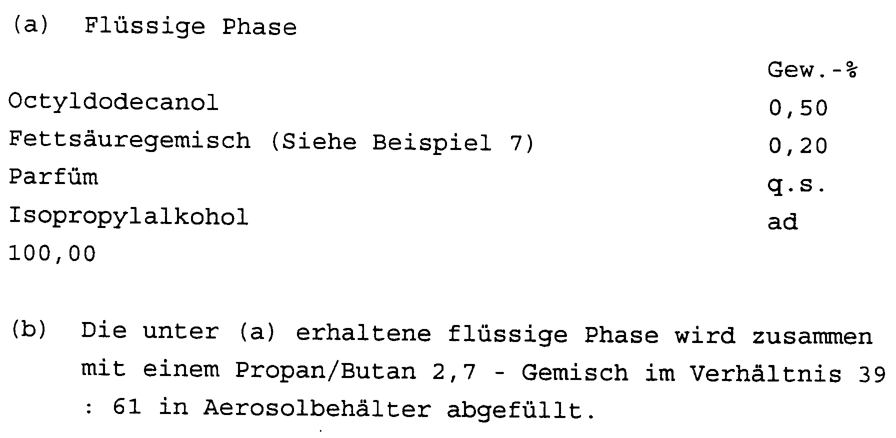

### Beispiel 8

### Pumpspray II

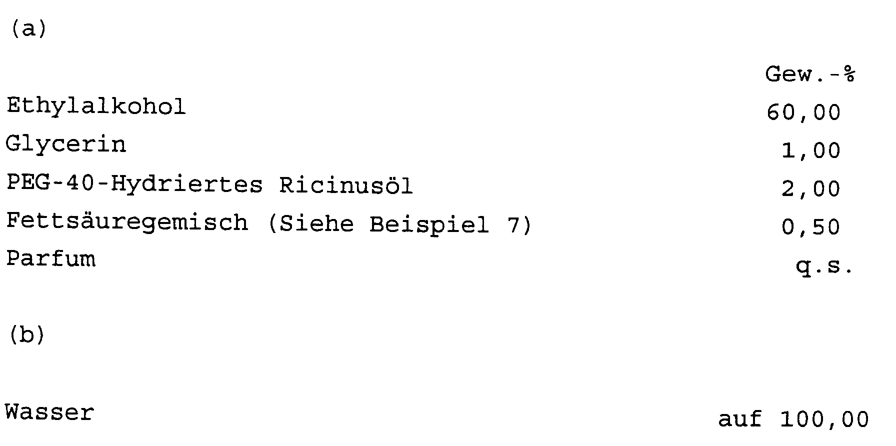

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 9

### Roll on - Gel II

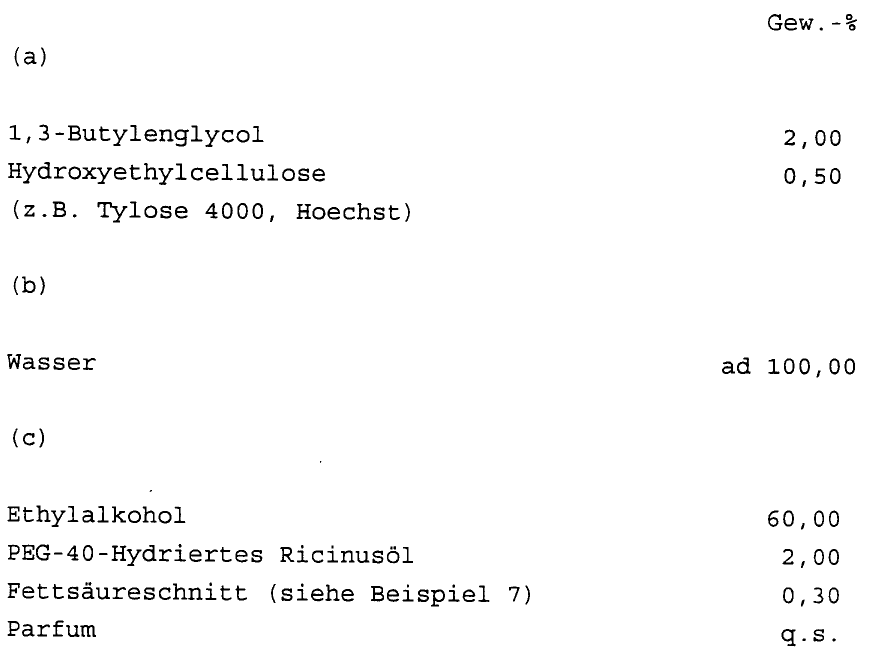

Die unter (a) genannten Bestandteile werden dispergiert, Wasser (b) wird zugegeben, bei Raumtemperatur quellen gelassen, eine Lösung der unter (c) genannten Bestandteile wird nach ca. 15 Minuten zugegeben. Die entstanden Mischung wird homogenisiert und kann abgefüllt werden.

### Beispiel 10

### Wachsstift II

| | Gew.-% |
|---|---|
| Hydriertes Ricinusöl | 5,00 |
| Bienenwachs | 6,00 |
| Ceresin (Hart-Ozokerit) | 30,00 |
| C₁₂₋₁₅-Alkohol-Benzoate | 17,00 |
| Fettsäuregemisch (siehe Beispiel 7) | 0,40 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 11

### Roll on - Emulsion II

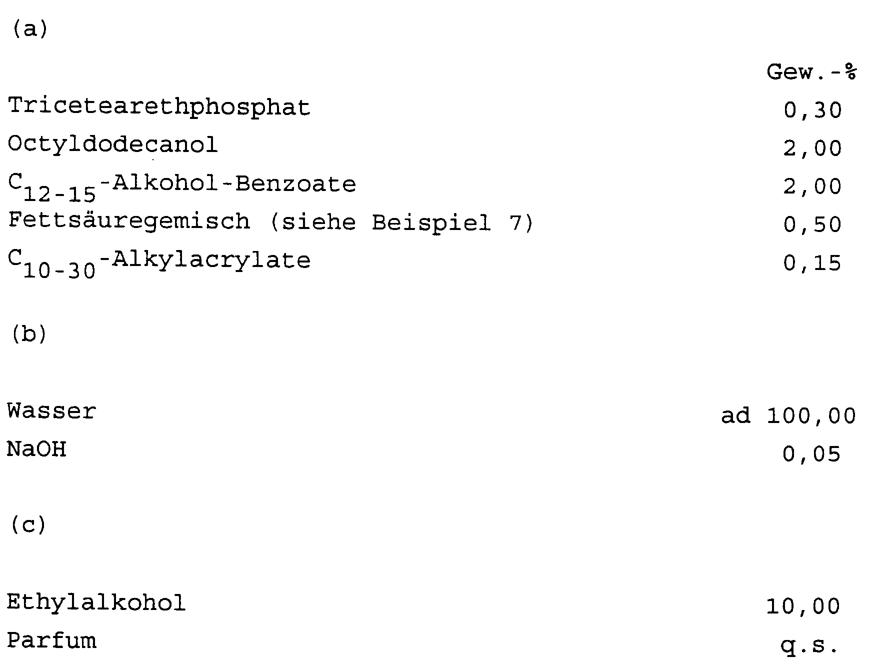

Die unter (a) und (b) genannten Bestandteile werden jeweils unter Rühren auf 75° C erwärmt. Sodann werden die Bestandteile (a) zu (b) gegeben. Die Mischung wird auf 35° C abgekühlt. Aus den Bestandteilen (c) wird eine Lösung hergestellt, welche auf 35° C erwärmt wird und unter Rühren zur Mischung aus (a) und (b) gegeben wird.

### Beispiel 12

### Pumpspray III

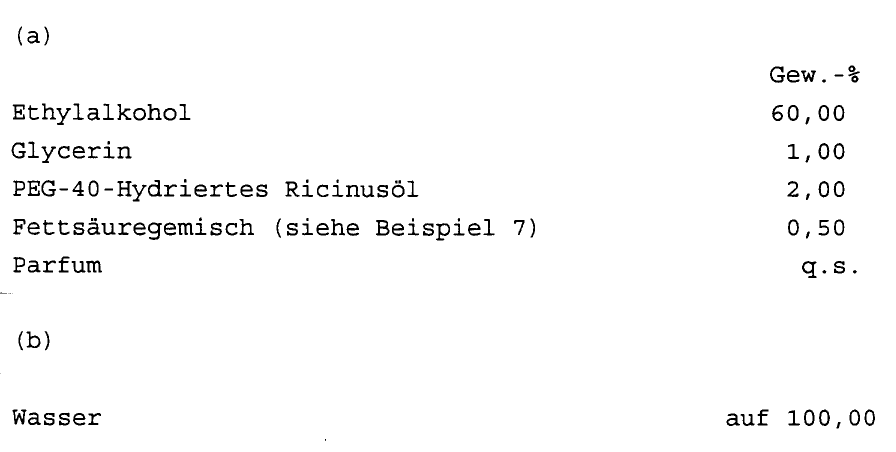

Die unter (a) genannten Bestandteile werden zu einer homogenen Lösung verarbeitet, sodann langsam mit der Wasserphase (b) aufgefüllt. Der fertige Pumpspray kann sodann in Pumpzerstäuber abgefüllt werden.

### Beispiel 13

### Roll on - Gel III

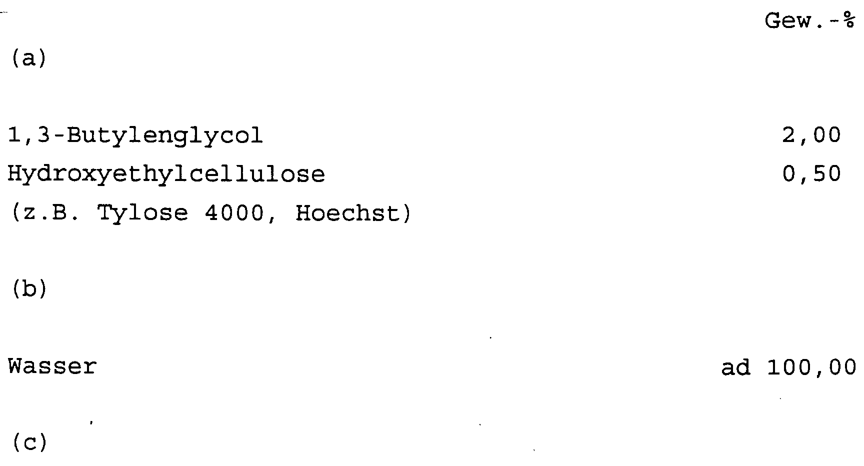

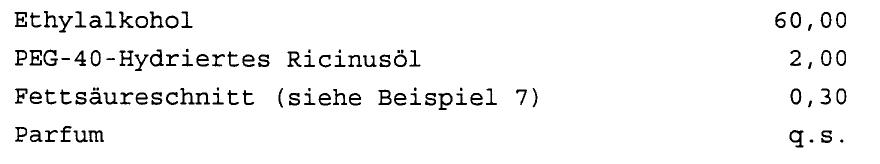

Die unter (a) genannten Bestandteile werden dispergiert, Wasser (b) wird zugegeben, bei Raumtemperatur quellen gelassen, eine Lösung der unter (c) genannten Bestandteile wird nach ca. 15 Minuten zugegeben. Die entstanden Mischung wird homogenisiert und kann abgefüllt werden.

### Beispiel 14

### Wachsstift III

| | Gew.-% |
|---|---|
| Hydriertes Ricinusöl | 5,00 |
| Bienenwachs | 6,00 |
| Ceresin (Hart-Ozokerit) | 30,00 |
| C₁₂₋₁₅-Alkohol-Benzoate | 17,00 |
| Fettsäuregemisch (siehe Beispiel 7) | 0,40 |
| Parfum | q.s. |
| Octyldodecanol | ad 100,00 |

Die Bestandteile werden bei ca. 75° C aufgeschmolzen, gut vermischt und in geeignete Formen gegossen.

### Beispiel 15

### Roll on - Emulsion III

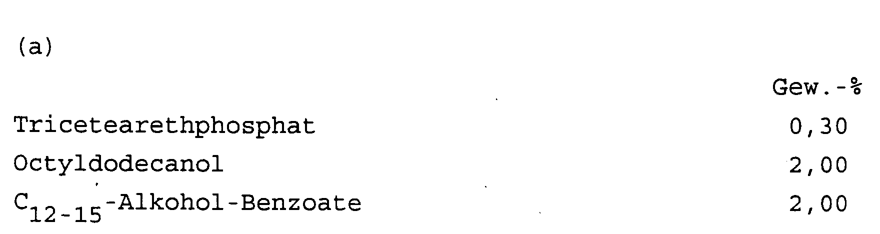

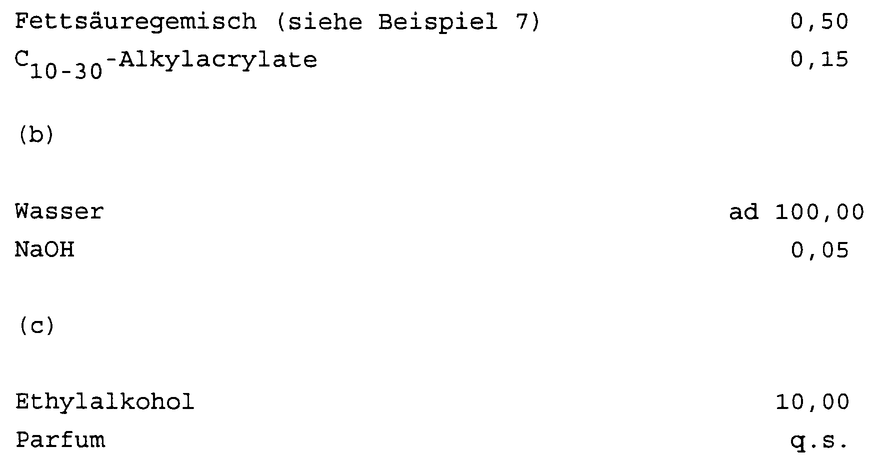

Die unter (a) und (b) genannten Bestandteile werden jeweils unter Rühren auf 75° C erwärmt. Sodann werden die Bestandteile (a) zu (b) gegeben. Die Mischung wird auf 35° C abgekühlt. Aus den Bestandteilen (c) wird eine Lösung hergestellt, welche auf 35° C erwärmt wird und unter Rühren zur Mischung aus (a) und (b) gegeben wird.

## Patentansprüche

1. Verwendung eines Gemisches aus
(a) Dodecansäure (Laurinsäure),
(b) mindestens einer weiteren Fettsäure, gewählt aus der Gruppe der unverzweigten gesättigten Fettsäuren einer Kettenlänge von C₆ ₋ ₂₀, wobei mindestens eine der weiteren Fettsäuren eine Kettenlänge größer als C₁₂ besitzen muß,
(c) Glycerylmonocaprinat und/oder Glycerylmonocaprinat
(d) unter Verzicht auf ethoxylierte Glycerylfettsäureester und propoxylierte Glycerylfettsäureester
(e) im pH-Bereich unter 8 vorliegend.
als Wirkprinzip zur selektiven Reduzierung coryneformer Bakterien.

2. Verwendung eines Gemisches aus
(a) Dodecansäure (Laurinsäure),
(b) mindestens einer weiteren Fettsäure, gewählt aus der Gruppe der unverzweigten gesättigten Fettsäuren einer Kettenlänge von C₆ ₋ ₂₀, wobei mindestens eine der weiteren Fettsäuren eine Kettenlänge größer als C₁₂ besitzen muß,
(c) Glycerylmonocaprinat und/oder Glycerylmonocaprinat
(d) unter Verzicht auf ethoxylierte Glycerylfettsäureester und propoxylierte Glycerylfettsäureester
(e) im pH-Bereich unter 8 vorliegend.
als Deowirkstoff.

3. Verwendung nach Anspruch 1 oder 2, dadurch gekennzeichnet, daß die weiteren Fettsäuren (b) aus der Gruppe Capronsäure (Hexansäure), Caprylsäure (Octansäure), Pelargonsäure (Nonansäure), Caprinsäure (Decansäure), Myristinsäure (Tetradecansäure), Palmitinsäure (Hexadecansäure) und Stearinsäure (Octadecansäure)
gewählt werden.

4. Verwendung nach Anspruch 1 oder 2, dadurch gekennzeichnet, daß folgende Gewichtsverhältnisse zwischen Laurinsäure und wenigstens einer weiteren Fettsäure gewählt werden, unabhängig, ob ein Gemisch der Laurinsäure mit einer oder mehreren weiteren Fettsäuren vorliegt:
Laurinsäure : Capronsäure = 50 : 0 bis 50 : 5 und/oder
Laurinsäure : Caprylsäure = 50 : 1 bis 50 : 20 und/oder
Laurinsäure : Pelargonsäure = 50 : 1 bis 50 : 20 und/oder
Laurinsäure : Caprinsäure = 50 : 1 bis 50 : 20 und/oder
Laurinsäure : Myristinsäure = 50 : 5 bis 50 : 30 und/oder
Laurinsäure : Stearinsäure = 50 : 1 bis 50 : 50,

5. Verwendung nach Anspruch 1 oder 2, dadurch gekennzeichnet, daß folgende Gewichtsverhältnisse zwischen Laurinsäure und wenigstens einer weiteren Fettsäure gewählt werden, unabhängig, ob ein Gemisch der Laurinsäure mit einer oder mehreren weiteren Fettsäuren vorliegt:
Laurinsäure : Capronsäure = 50 : 0 bis 50 : 1 und/oder
Laurinsäure : Caprylsäure = 50 : 2 bis 50 : 15 und/oder
Laurinsäure : Pelargonsäure = 50 : 2 bis 50 : 15 und/oder
Laurinsäure : Caprinsäure = 50 : 2 bis 50 : 15 und/oder
Laurinsäure : Myristinsäure = 50 : 10 bis 50 : 20 und/oder
Laurinsäure : Stearinsäure = 50 : 2 bis 50 : 20.

6. Verwendung nach Anspruch 1 oder 2, dadurch gekennzeichnet, daß folgende Gewichtsverhältnisse zwischen Laurinsäure und wenigstens zwei weiteren Fettsäure gewählt werden, unabhängig, ob ein Gemisch der Laurinsäure mit zwei oder noch mehr weiteren Fettsäuren vorliegt:
Laurinsäure : Caprylsäure : Caprinsäure = 50 : 1 : 1 bis 50 : 20 : 20,
bevorzugt
Laurinsäure : Caprylsäure : Caprinsäure = 50 : 1 : 1 bis 50 : 5 : 5.

7. Verwendung nach Anspruch 1 oder 2, durch folgende Zusammensetzung der erfindungsgemäßen Fettsäuregemische, jeweils bezogen auf das Gesamtgewicht des Fettsäuregemisches, gekennzeichnet:
| | |
|---|---|
| Laurinsäure | 10 - 90 Gew.-% |
| Myristinsäure | 13 - 18 Gew.-% |
| Palmitinsäure | 8 - 10 Gew.-% |
| Caprylsäure | 6 - 9 Gew.-% |
| Caprinsäure | 6 - 10 Gew.-% |
| Stearinsäure | 10 - 90 Gew.-% |
| Capronsäure | 0 - 1 Gew.-%. |

8. Verwendung nach Anspruch 1 oder 2, dadurch gekennzeichnet, daß ein Gehalt an GMCy und/oder GMC vorteilhaft 0,1 - 10,0 Gew.-%, bevorzugt 0,5 bis 7,5 Gew.-%, besonders bevorzugt 1,5 - 5,0 Gew.-%, jeweils bezogen auf das Gesamtgewicht der jeweiligen Formulierung, gewählt wird.

9. Verwendung nach Anspruch 1 oder 2, dadurch gekennzeichnet, daß das Verhältnis von GMCy und/oder GMC einerseits und dem Fettsäuregemisch andererseits zueinander aus dem Bereich von 20 : 1 bis 1 : 20 gewählt werden, bevorzugt aus dem Bereich von 5 : 1 bis 1 : 5, gewählt wird.
